# FASCICULE DE BREVET EUROPEEN

(11) **EP 1 747 731 B1**
(45) Date de publication et mention de la délivrance du brevet: **19.01.2011**
(21) Numéro de dépôt: 06014194.2
(22) Date de dépôt: 08.07.2006
(51) Int. Cl.: A41D 13/00, A41D 31/02, A41B 17/00, A41B 11/14

(54) **Vêtement ajusté**
Dichtanliegendes Bekleidungsstück
Close-fitting garment

(30) Priorité: 29.07.2005 FR 0508103
(43) Date de publication de la demande: 31.01.2007
(60) Demande divisionnaire: 10012354.6
(73) Titulaire: SALOMON S.A.S., 74370 Metz-Tessy (FR)
(72) Inventeur: Chapuis, Serge, 73610 Lepin Le Lac (FR); Roux-Govaert, Isabelle, 38180 Seyssins (FR)
(74) Mandataire: Rambaud, Pascal

(56) Documents cités:
- EP-A- 0 705 543
- EP-A- 1 016 351
- DE-A1-102004 034 699
- US-A- 5 154 690
- PATENT ABSTRACTS OF JAPAN vol. 013, no. 515 (C-655), 17 novembre 1989 (1989-11-17) & JP 01 207403 A (HARUMATSUKU:KK; others: 01), 21 août 1989 (1989-08-21)

## Description

La présente invention concerne un vêtement, notamment de sport, de type ajusté et conçu pour être porté sur une partie supérieure et/ou inférieure du corps.

De tels vêtements sont couramment utilisés, par exemple sous forme de collants portés sur la partie inférieure du corps pour la pratique de la course à pied.

Il est connu par exemple par le US 5 367 708 de concevoir des parties en matériau moins extensible que d'autres dans des zones déterminées du collant, ces parties moins extensibles couvrant certains muscles, mais pas d'autres. Ce document décrit également un tel principe adapté à des vêtements de type tee-shirt ou chaussettes. Ces vêtements connus sont en fait conçus pour exercer un effet de bandage, ayant une fonction support des muscles et articulations concernés de façon à éviter les blessures.

Par le EP 1 250 858, il est connu de réaliser des vêtements près, du corps, constitués de panneaux élastiques reliés par des coutures, les coutures étant disposées de façon à réduire l'extensibilité des panneaux et augmenter l'effet de retenue/contention. Les vêtements sont très près du corps et extrêmement difficiles à enfiler, en effet tout glissement relatif du vêtement par rapport à la peau de l'utilisateur doit être évité.

Par le WO 98/36652 il est connu de réaliser des vêtements ajustés constitués d'un tissu de base élastique tel que du lycra ® sur lequel sont cousues des bandes de résistance élastique. Ces bandes élastiques exercent une résistance élastique au mouvement de façon à aider à la musculation du corps.

Le document, EP 1 016 351, montre un collant ayant des bandes de renfort constituées d'un lamine selon le prèambule de la revendication 1.

II est également connu par le FR 2 548 892 et le FR 2 597 123 de concevoir des articles de contention à usage médical ou sportif, constitués de fils élastiques tricotés selon un processus spécial pour obtenir le degré de contention souhaité. De tels articles sont destinés à améliorer la circulation du sang.

Le EP 0 934 043 concerne également un bas de contention constitué de fils élastiques tricotés exerçant une contention plus importante au niveau du mollet qu'au niveau du pied et destinés plus spécifiquement à l'activité sportive.

Ce bas est porté pendant l'effort et après l'effort de façon à améliorer la récupération, surtout en cas de transport prolongé ou de piétinement après l'effort. En améliorant la circulation sanguine, ces bas de contention permettent de réduire fortement les crampes, dysesthésie et la lourdeur et fatigabilité des jambes et facilitent la récupération sanguine.

Un but de la présente invention est de proposer un vêtement ajusté permettant d'améliorer la circulation sanguine et de faciliter la récupération après un effort.

Un autre but de la présente invention est de proposer un vêtement ajusté permettant un effet de massage.

Un but de la présente invention est également de fournir une autre conception de vêtement de sport ajusté, permettant notamment d'en faciliter la fabrication et/ou d'en améliorer l'esthétique.

Ce but est atteint dans la présente invention par le fait que le vêtement de sport selon la revendication 1 est constitué principalement d'un laminé textile extensible/film élastique dans au moins une zone du vêtement entourant une zone du corps.

Le matériau textile peut être un textile classique type jersey, maille avec ou sans fibres élastiques tel que l'élasthanne, lycra®, spandex, PBT, PET, tandis que le film élastique sera de préférence un film de type PU tel qu'un film TEKFILM® de type Tradel, c'est-à-dire en un matériau ayant de grandes caractéristiques d'élasticité.

La combinaison textile extensible/film élastique permet d'obtenir un vêtement très élastique pouvant stimuler, supporter et/ou masser l'ensemble des muscles, ainsi qu'améliorer la circulation sanguine et lymphatique pour l'exercice d'une pression de contention.

Par ailleurs la structure laminée permet de faire varier très simplement le degré d'élasticité du vêtement en faisant varier simplement l'épaisseur, le type ou le degré de recouvrement du film élastique sur une surface donnée.

La structure laminée permet, par son approche modulaire, de changer très facilement la fabrication puisqu'un simple changement du film ou de la découpe de celui-ci permet, pour un même patronage du tissu de base, de changer les caractéristiques de l'ensemble du vêtement.

La fabrication est beaucoup plus simple et souple puisqu'elle ne nécessite pas de réglages longs, délicats et fastidieux de machines de tricotage comme pour les bas de contention connus.

Par ailleurs le collage du film élastique, au lieu de l'assemblage par couture comme dans le WO 98/36652 permet un meilleur confort du vêtement, sans création "d'effet ficelle", inconfortable.

La colle utilisée pour la structure laminée participe également à la "raideur élastique" ou degré de contention de l'ensemble

Le film élastique peut être laminé sous une forme continue c'est-à-dire sans discontinuité. Selon un mode de réalisation le film est prédécoupé avant laminage, c'est-à-dire collage, de façon à constituer des zones d'aération sur le tissu.

Ces découpes pourront être effectuées selon un schéma répétitif et régulier de façon à envelopper et stimuler les muscles de manière homogène et uniforme. Les découpes pourront au contraire être selon un motif répétitif asymétrique de façon à donner au vêtement des raideurs ou pressions de contention plus importantes selon des directions déterminées.

La structure laminée permet donc également de modifier très facilement l'esthétique du vêtement en changeant simplement le motif des découpes du film.

De toute façon l'invention sera mieux comprise et d'autres caractéristiques de celle-ci seront mises en évidence à l'aide de la description qui suit en référence au dessin schématique annexé dans lequel :
- la figure 1 est une vue de devant d'un collant selon l'invention,
- la figure 2 est une vue de côté du collant de la figure 1,
- la figure 3 est une vue de derrière du collant de la figure 1,
- la figure 4 illustre un premier motif de découpe du film élastique,
- la figure 5 illustre un second motif de découpe du film élastique,
- la figure 6 est une vue en coupe selon VI VI de la figure 3,
- la figure 7 est une vue similaire à la figure 6 illustrant un autre mode de réalisation,
- la figure 8 est une vue similaire à la figure 6 illustrant un autre mode de réalisation,
- la figure 9 est une vue de devant d'un tee-shirt selon l'invention,
- la figure 10 est une vue de derrière du tee-shirt de la figure 9.

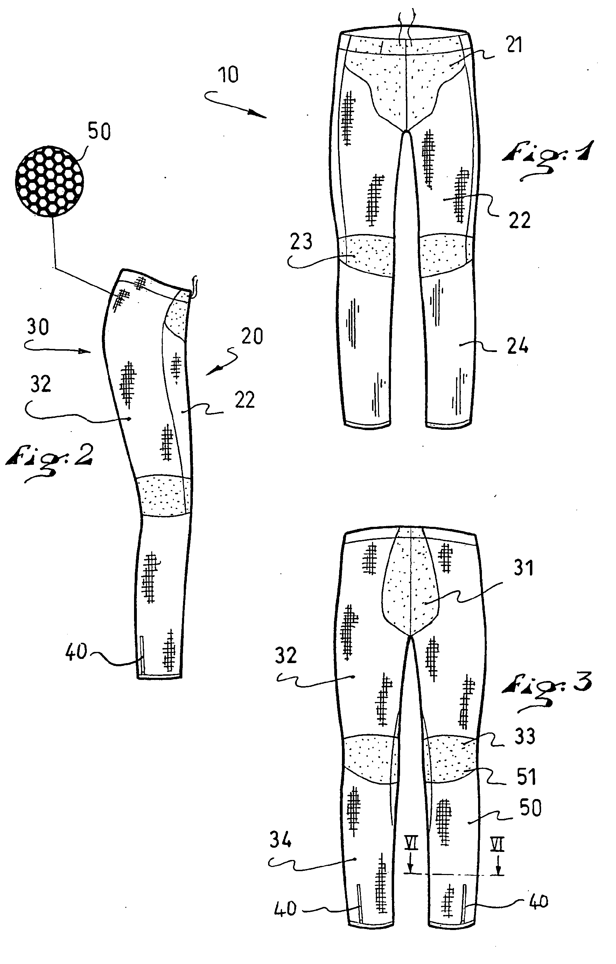

Les figures 1 à 3 illustrent l'application de la présente invention à un collant 10 destiné notamment à la course à pied. Ce collant présente une partie "devant" 20 représentée sur la figure 1 et une partie "derrière" 30 représentée sur la figure 3.

Ainsi que le montrent ces figures, ce collant 10 est constitué sur la majeure partie de sa surface, à la fois devant 20 et derrière 30, d'un laminé 50 selon l'invention.

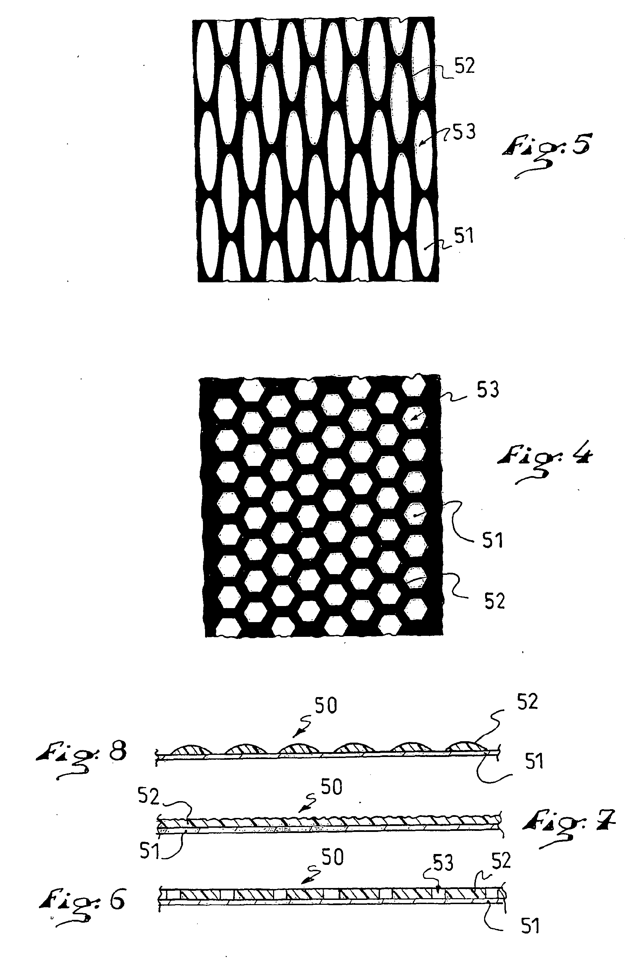

Ainsi que le montre la figure 6 le laminé 50 est constitué d'une première couche de matériau textile 51 et d'une seconde couche de film élastique 52, ces deux couches étant contrecollées.

Le matériau textile est un matériau de type jersey, maille, ... en coton, polyester, polyamide, c'est-à-dire toute maille extensible. Il peut également être constitué d'un tissu comportant des fibres élastiques telles qu'élasthanne, lycra®, spandex, PBT, PET. Ce matériau textile est un tissu extensible du fait de sa construction (jersey), ou des fibres le constituant. Il est plus ou moins élastique.

Selon un mode de réalisation il est en matériau aéré de type maille, filet ("mesh") et/ou évacuant l'humidité et/ou bactéricide tel que du X-Static®.

Le film élastique 52 est par exemple un film PU tel que vendu sous la dénomination commerciale Tradel ou Tekfilm® de Framis, ou un film PU vendu par Bemis. Ce peut être également du PVC ou silicone.

Ces films PU sont obtenus par enduction de couches de polyéruthane superposées, la stratification permettant d'obtenir une surface adhésive.

Le film élastique de PU, PVC, silicone peut également être un dépôt direct sans adhésif supplémentaire, par exemple par enduction ou sérigraphie. Le film peut être collé sur le tissu par pressage à chaud ou être fourni directement laminé sur le tissu.

Dans l'exemple représenté le film élastique est un film PU. Il est prédécoupé avant collage et comporte donc des découpes ou ouvertures 53.

Ces découpes 53 peuvent avoir une forme polygonale, en l'occurrence hexagonale, comme représenté sur les figures 1 à 3 et 4. Cette forme polygonale peut être régulière ou symétrique de façon que la contrainte élastique exercée par le film PU soit régulière.

La forme polygonale peut également être asymétrique, et par exemple rectangulaire pour que la contrainte élastique ne soit pas uniforme.

Les découpes 53 peuvent également avoir une forme courbe, symétrique par exemple circulaire, ou asymétrique par exemple en forme d'ellipse comme représentée sur la figure 5.

Les découpes 53 permettent de conférer à l'ensemble du vêtement une bonne respirabilité, aération, puisque le tissu de base 51 n'est alors pas recouvert et apparaît à travers celles-ci.

Si une telle respirabilité n'est pas souhaitée, par exemple pour un vêtement d'hiver, il peut être prévu que le film 52 soit uniforme, et donc sans découpes, comme représenté sur la figure 7.

Le film PU 52 peut également être déposé sous forme de plots 52 (cf. figure 8), de formes hémisphériques ou autres qui font saillie par rapport à la couche de textile 51 et peuvent donc avoir un effet massage. L'effet massage est également obtenu avec les découpes du fait de glissement relatif du vêtement par rapport à la peau de l'utilisateur.

Dans ce cas les plots 52 seront plutôt positionnés à l'intérieur du vêtement, ils pourront cependant être aussi à l'extérieur.

Dans tous les cas le laminé fournit également un effet esthétique/décoratif très appréciable. Cet effet pourra être renforcé par des effets de couleur (en utilisant par exemple un film élastique de couleur contrastée par rapport au textile support), d'impression (en couleur et/ou en relief) du film élastique; en jouant également sur les épaisseurs et/ou relief du film élastique, etc. le film élastique pourrait également servir à coller un autre tissu prédécoupé de la même façon pour un effet esthétique particulier. Dans ce cas le film élastique est un film de collage double adhésion.

De façon similaire, les motifs de découpe pourront être réguliers ou irréguliers pour des raisons également esthétiques.

Dans tous les cas le patronage du vêtement, au moins dans les zones de contention, correspond à des dimensions inférieures à celles du corps de l'utilisateur de façon que le tissu soit mis sous tension lors de l'enfilage et puisse exercer l'action de contention élastique/massage souhaitée.

Des essais de traction à l'aide d'une machine de traction de type Adamel Lhomargy montrent que le simple collage film PU sur un matériau plus ou moins extensible permet d'augmenter notablement (en l'occurrence multiplier par 3 dans l'exemple ci-dessous), la résistance élastique du tissu. Le tableau ci-dessous indique les valeurs d'effort de traction en N/an devant être exercées pour un tissu recouvert ou non de film élastique.

| Tissu | Seul | Avec film élastique Tradel® |
|---|---|---|
| Power lycra® | 1,44 | 4,5 |
| Malaga lycra® | 1,26 | 4, 75 |

Comme le montrent également les figures 1 à 3, le laminé sera interrompu dans les zones du vêtement correspondant aux parties les plus thermogènes du corps. Dans l'exemple représenté sur les figures 1 à 3, ces zones les plus thermogènes et donc dépourvues de film élastique sont les zones d'articulation des genoux respectivement avant 23 et arrière 33, et les zones du bassin 21 et postérieur 31 et ne comportent donc que la couche textile 51. Elles peuvent également être en un autre matériau textile plus aéré. Les parties supérieures avant 22 et arrière 32, les parties tibias 24 et mollets 34 sont donc complètement recouvertes par le laminé 50 selon l'invention, en d'autres termes la cuisse et la jambe sont complètement entourées ou enveloppées par le laminé.

Des fermetures à glissières 40 sont prévues à l'extrémité inférieure des mollets 34 pour permettre l'ouverture du collant dans le bas des jambes et faciliter son enfilage. Ces fermetures peuvent également être prévues sur le devant du vêtement et/ou remplacées pars d'autres types de fermetures à pression, bouton, etc.

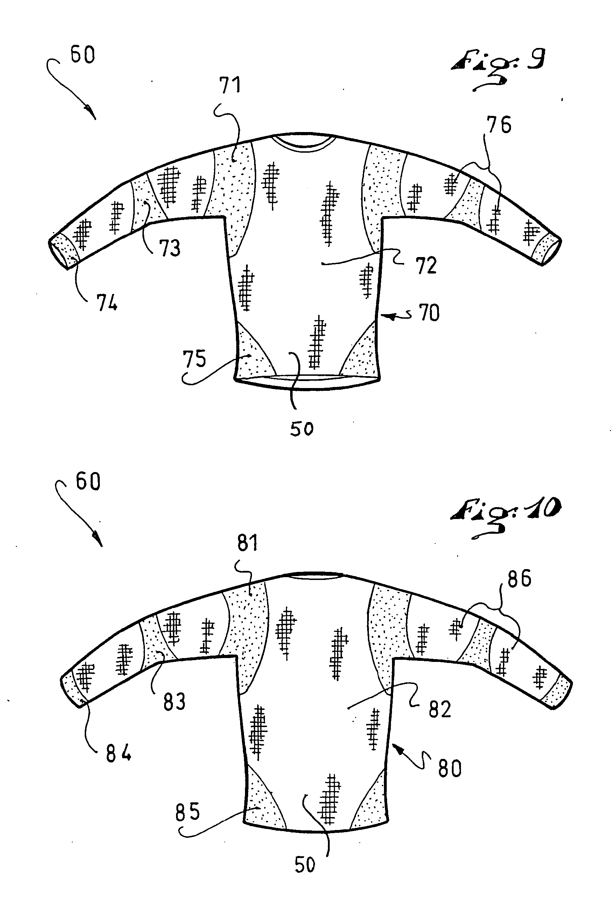

Les figures 9 et 10 illustrent l'application de l'invention à un tee-shirt 60 de type manches longues.

Dans ce cas le laminé 50 couvre et enveloppe complètement le devant 72 et le dos 82 du buste ainsi que les zones respectivement avant 76 et arrière 86 des bras et avant bras.

De même que dans le cas précédent le laminé 50 est interrompu dans les zones du vêtement correspondant aux zones les plus thermogènes du corps, en l'occurrence les coudes 73, 83, les zones d'aisselle/articulation d'épaule 71, 81, les zones de poignet 74, 84 et les parties latérales de la taille 75, 85 et est remplacé dans ces zones par un matériau textile plus aéré.

La présente invention n'est pas limitée aux seuls exemples de réalisation décrits ci-avant à titre d'exemples non limitatifs et peut également être appliquée à d'autres vêtements et notamment tee-shirt à manches courtes, maillot, sans que l'on sorte pour autant du cadre de la présente invention.

Le laminage de film élastique peut également n'être effectué que sur certaines parties du vêtement selon le degré de contention, et/ou technicité du vêtement recherché sur les parties correspondantes du corps.

Par exemple dans le cas d'un collant, on peut envisager de n'avoir l'effet contention que dans la zone mollet où l'effet contention est important pour la circulation sanguine et le laminage ne sera donc effectué que sur la jambe c'est-à-dire la partie s'étendant du genou au pied. On peut également souhaiter n'avoir de la contention ou un effet massage que dans les zones de la cuisse, etc.

## Revendications

1. Vêtement de sport ajusté pour l'exercice d'une pression de contention constitué principalement d'un laminé ; ledit laminé étant constitué d'une première couche d'un matériau textile extensible (51) et d'une deuxième couche de film élastique (52) dans au moins une zone (22,32 ; 24,34 ; 72,82 ; 76,86) du vêtement,
**caractérisé en ce que** le film (52) comprend des découpes (53) qui forment un schéma répétitif , le laminé étant interrompu dans les zones du vêtement correspondant aux zones les plus thermogènes du corps
et **en ce que** le vêtement est soit du type collant et la cuisse ou la jambe sont entourées complètement par le laminé, ou bien le vêtement est du type tee-shirt à manches longues, le laminé (50) couvrant et enveloppant complètement le devant (72) et le dos (82) du buste ainsi que les zones respectivement avant (76) et arrière (86) des bras et avant bras.

2. Vêtement de sport selon la revendication 1 **caractérisé en ce que** le laminé est interrompu dans les zones d'articulation des genoux, des coudes, des zones d'aisselle et des zones de poignets.

3. Vêtement de sport selon l'une des revendications 1 à 3, **caractérisé en ce que** le film élastique (52) est un film PU, PVC, silicone.

4. Vêtement de sport selon l'une des revendications 1 à 4, **caractérisé en ce que** le film élastique (52) est un film continu.

5. Vêtement de sport selon l'une des revendications 1 ou 5, **caractérisé en ce que** les découpes (53) délimitent des trous polygonaux.

6. Vêtement de sport selon l'une des revendications 1 ou 5, **caractérisé en ce que** les découpes (53) délimitent des trous hexagonaux.

7. Vêtement de sport selon l'une des revendications 1 à 5, **caractérisé en ce que** les découpes (53) délimitent des trous de forme courbe.

8. Vêtement de sport selon l'une des revendications 1 à 8, **caractérisé en ce que** le matériau textile (51) est aéré.

9. Vêtement de sport selon l'une des revendications 1 à 8, **caractérisé en ce qu'**il s'agit d'un collant et **en ce que** le laminé couvre au moins la partie jambe (24, 34) de ce collant.

10. Vêtement de sport selon l'une des revendications 1 à 9, **caractérisé en ce qu'**il s'agit d'un collant et **en ce que** le laminé couvre au moins la partie cuisse (24, 34) de ce collant.

## Claims

1. Sports garment adjusted for exerting a restraining pressure, mainly comprised of a laminate; said laminate being comprised of a first layer of stretchable textile material (51) and of a second layer of elastic film (52) in at least one area (22, 32; 24, 34; 72, 82; 76, 86) of the garment,
**characterized in that** the film (52) includes cutouts (53) that form a repetitive scheme, the laminate being interrupted in the areas of the garment corresponding to the most heat-generating areas of the body,
and **in that** the garment is either a pair of tights, and the thigh or the leg are completely surrounded by the laminate, or a long-sleeved tee-shirt, the laminate (50) completely covering and enveloping the front (72) and back (82) of the bust, as well as the front (76) and rear (86) zones, respectively, of the arms and forearm.

2. Sports garment according to claim 1, **characterized in that** the laminate is interrupted in the articulation zones of the knees, elbows, as well as of the armpits and wrists.

3. Sports garment according to one of claims 1 to 3, **characterized in that** the elastic film (52) is a PU, PVC, silicone film.

4. Sports garment according to one of claims 1 to 4, **characterized in that** the elastic film (52) is a continuous film.

5. Sports garment according to one of claims 1 or 5, **characterized in that** the cutouts (53) demarcate polygonal holes.

6. Sports garment according to one of claims 1 or 5, **characterized in that** the cutouts (53) demarcate hexagonal holes,

7. Sports garment according to one of claims 1 or 5, **characterized in that** the cutouts (53) demarcate curved holes.

8. Sports garment according to one of claims 1 to 8, **characterized in that** the textile material (51) is ventilated.

9. Sports garment according to one of claims 1 to 8, **characterized in that** it is a pair of tights, and **in that** the laminate covers at least the leg portion (24, 34) of this pair of tights.

10. Sports garment according to one of claims 1 to 9, **characterized in that** it is a pair of tights, and **in that** the laminate covers at least the thigh portion (24, 34) of this pair of tights.

## Patentansprüche

1. Sportbeldeidung, eingestellt zum Ausüben eines Kompressionsdrucks, welche hauptsächlich aus einem Laminat besteht, wobei das Laminat aus einer ersten Lage eines dehnbaren textilen Materials (51) und einer zweiten Lage aus einer elastischen Schicht (52) in zumindest einem Bereich (22, 32; 24, 34; 72, 82; 76, 86) des Kleidungsstücks besteht,
**dadurch gekennzeichnet, dass** die Schicht (52) Formschnitte (53) umfasst, die ein sich wiederholendes Schema bilden, wobei das Laminat in den Bereichen des Kleidungsstücks unterbrochen ist, die den Bereichen des Körpers entsprechen, die am meisten Wärme entwickeln,
und **dadurch**, dass das Kleidungsstück entweder strumpfhosenähnlich ist und der Oberschenkel oder das Bein vollständig von dem Laminat umgeben sind, oder aber das Kleidungsstück einem T-Shirt mit langen Ärmeln ähnlich ist, wobei das Laminat (50) vollständig die Vorderseite (72) und die Rückseite (82) der Brust und folglich auch die jeweiligen vorderen (76) und hinteren (86) Bereiche der Arme und der Unterarme abdeckt und umhüllt.

2. Sportbeldeidung nach Anspruch 1, **dadurch gekennzeichnet, dass** das Laminat in den Bereichen der Kniegelenke, der Ellbogen, der Achselbereiche und der Handgelenkbereiche unterbrochen ist.

3. Sportbekleidung nach einem der Ansprüche 1 und 2, **dadurch gekennzeichnet, dass** die elastische Schicht (52) eine PU-, PVC-, Silikonschicht ist.

4. Sportbekleidung nach einem der Ansprüche 1 bis 3, **dadurch gekennzeichnet, dass** die elastische Schicht (52) eine durchgehende Schicht ist.

5. Sportbekleidung nach einem der Ansprüche 1 bis 4, **dadurch gekennzeichnet, dass** die Formschnitte (53) polygonale Löcher begrenzen.

6. Sportbekleidung nach einem der Ansprüche 1 bis 4, **dadurch gekennzeichnet, dass** die Formschnitte (53) hexagonal Löcher begrenzen.

7. Sportbekleidung nach einem der Ansprüche 1 bis 4, **dadurch gekennzeichnet, dass** die Formschnitte (53) kurvenförmige Löcher begrenzen.

8. Sportbekleidung nach einem der Ansprüche 1 bis 7, **dadurch gekennzeichnet, dass** das Textilmaterial (51) belüftet ist.

9. Sportbekleidung nach einem der Ansprüche 1 bis 8, **dadurch gekennzeichnet, dass** es sich um eine Strumpfhose handelt, und dass das Laminat zumindest den Beinteil (24, 34) dieser Strumpfhose bedeckt.

10. Sportbekleidung nach einem der Ansprüche 1 bis 9, **dadurch gekennzeichnet, dass** es sich um eine Strumpfhose handelt und dass das Laminat zumindest den Oberschenkelteil (24,34) dieser Strumpfhose bedeckt.
